(19) Europäisches Patentamt
European Patent Office
Office européen des brevets

(11) **EP 3 418 717 A1**

(12) **EUROPEAN PATENT APPLICATION**

(43) Date of publication:
26.12.2018 Bulletin 2018/52

(51) Int Cl.:
*G01N 15/10* (2006.01)   *C12N 5/071* (2010.01)
*B01L 3/00* (2006.01)   *G01N 15/14* (2006.01)

(21) Application number: 17177624.8

(22) Date of filing: 23.06.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Cellix Limited**
**12 Dublin (IE)**

(72) Inventors:
• **Kashanin, Dmitry**
**Dublin, 12 (IE)**
• **Shvets, Igor**
**Dublin, 12 (IE)**
• **Mc Auley, Robert**
**Navan, Co. Meath C15 Y2D7 (IE)**

(74) Representative: **Purdy, Hugh Barry**
**PurdyLucey**
**Intellectual Property Limited**
**6-7 Harcourt Terrace**
**Dublin D02P210 (IE)**

(54) **A MICROFLUIDIC APPARATUS FOR SEPARATION OF PARTICULATES IN A FLUID**

(57)    A microfluidic apparatus for separation of particulates in a fluidAn apparatus for separation of particulates in a fluid (3A) into subsets of particulates comprises a microfluidic chip (1) comprising: a microfluidic channel having a fluid inlet for receipt of a stream of particulate containing fluid (3A), a detection zone disposed in the microfluidic channel and comprising a sensor configured to detect changes in the microfluidic channel corresponding to particulates passing the sensor, and a separation zone distal of the detection zone in which the microfluidic channel divides into at least two secondary microfluidic channels (4). The separation zone comprises two or more separation electrodes (16A, 16B) including at least one separation electrode (16A, 16B, 16C, 16D) disposed in electrical contact with an interior of the microfluidic channel and at least one further separation electrode (16A, 16B, 16C, 16D) disposed in electrical contact with an interior of the microfluidic channel or one of the secondary microfluidic channels (4). The pair of separation electrodes (16A, 16B) are configured to pass a pulse of electrical current through the separation zone of the microfluidic channel.

Figure 1

**Description**

Field of the Invention

The present invention relates to an apparatus comprising a microfluidic chip useful for separating a particulate containing fluid into subsets of particulates. Also contemplated are methods of separating a particulate containing fluid into subsets of particulates, for examples subsets of cells. In particular, the invention relates to an on-chip microfluidic method of separating semen cells according to sex.

Background to the Invention

Most of the embodiments for the particle/cell separation described deal with a specific method of particles/cells identification based on AC impedance spectroscopy. We will therefore first review the technology of AC impedance spectroscopy while emphasising that the device and method for the particle/cell separation can also be used with other methods and devices for the particle/cell identification such as e.g. optical scattering or optical fluorescence method.

In recent years, microfluidic impedance cytometry has been further developed to count and discriminate between different kinds of cells. Multi-frequency impedance measurements can be used to determine the electrical properties of single cells in a microchip [S. Gawad, L. Schild, P.H. Renaud, Micromachined impedance spectroscopy flow cytometer for cell analysis and particle sizing, Lab. Chip. 2001 1 76-82].

Typical impedance measurement scheme described in preferred embodiments utilize detection of particles (cells) based on a lock-in amplifier measurement and AC impedance spectroscopy measurements. A lock-in amplifier is a type of amplifier that can extract a minute signal due to particle (cell) passing through the detection area from a noisy environment. Depending on the dynamic reserve of the instrument, signals up to one million times smaller than noise components and relatively close in frequency, can still be reliably detected and segregated. Lock-in amplifier is generally used to detect and measure very small AC signals, in the range of a few nanoVolts. The measured value is complex AC impedance between two electrodes separated by the fluid in the microchannel. The most important part of this amplifier is a digital low-pass filter, the part that isolates the frequency of interest and eliminates the higher frequencies and noise. In order to achieve a real-time analysis of the cells with the rate of up to many thousands of cells per second, the detection system must be very fast.

The amplifier is essentially a homodyne detector followed by low pass filter. Homodyne detector is a device that detects frequency-modulated signal by non-linear by mixing it with a signal having the same frequency as the modulation signal. Whereas conventional lock-in amplifiers use analogue frequency mixers and R-C filters for the demodulation, state of art instruments have both steps implemented by fast digital signal processing for example using Field Programmable Gate Array (FPGA). FPGA is an integrated digital circuit whose logic behaviour and component mapping is programmable. Sine and cosine demodulation is performed simultaneously, which is sometimes also referred to as dual phase demodulation.

Operation of a lock-in amplifier relies on the orthogonality of sinusoidal functions. When a sinusoidal function of frequency $f_1$ is multiplied by another sinusoidal function of frequency $f_2$ not equal to $f_1$ and integrated over a time much longer than the period of the two functions, the result is zero. Instead, when $f_1$ is equal to $f_2$ and the two functions are in phase, the average value is equal to half of the product of the amplitudes.

A lock-in amplifier takes the input signal, multiplies it by the reference signal (either provided from the internal oscillator or an external source), and integrates it over a specified time, usually on the order of milliseconds to a few seconds. The resulting signal is a DC signal, where the contribution from any signal that is not at the same frequency as the reference signal, is attenuated close to zero. The out-of-phase component of the signal that has the same frequency as the reference signal is also attenuated (because sine functions are orthogonal to the cosine functions of the same frequency), making a lock-in a *phase-sensitive detector* (PSD).

Usually, lock-in amplifiers generate their own internal reference signal by a phase-locked-loop (PLL) locked to the external reference. The internal reference is $V_L \sin(\omega_L t + \theta_{ref})$, where $\omega_L$ is the angular frequency of the signal and $\theta_{ref}$ is the phase of the reference signal.

The lock-in amplifies the signal and then multiplies it by the lock-in reference using a phase-sensitive detector or multiplier. The output of the PSD is simply the product of two sine waves.

$$V_{psd} = V_{sig}V_L \sin(\omega_r t + \theta_{sig}) \sin(\omega_L t + \theta_{ref}) =$$

$$= \frac{1}{2}V_{sig}V_L \cos([\omega_r - \omega_L]t + \theta_{sig} - \theta_{ref}) -$$

$$\frac{1}{2}V_{sig}V_L \cos([\omega_r + \omega_L]t + \theta_{sig} + \theta_{ref})(1.1)$$

The PSD output is two AC signals, one at the difference frequency $[\omega_r - \omega_L]$ and the other one at sum frequency $[\omega_r + \omega_L]$. Then the PSD output passes through a low pass filter and the AC signals are removed.

If $\omega_r$ equals $\omega_L$, the difference frequency component in eq. (1.1) will be a DC signal. So, the filtered PSD output will be:

$$V_{psd} = \frac{1}{2}V_{sig}V_L \cos(\theta_{sig} - \theta_{ref}) \qquad\qquad (1.2)$$

which is a DC signal proportional to the signal amplitude.

Unlike in analogue amplifiers, in a digital lock-in the signal and reference are converted into a digital form by Analogue-to-Digital converters (ADC) and are represented by sequences of numbers. Multiplication and filtering are performed mathematically by a digital signal processing (DSP). The phase sensitive detectors (PSDs) in the digital lock-in act as linear multipliers; that is, they multiply the signal by a reference sine wave. Analogue PSDs (both square wave and linear) have many problems associated with them such as output offsets and gain error. Output offset is a problem of analogue lock-ins because the signal of interest is a DC output from the PSD, and an output offset contributes to error and zero drift. The offset problems of analogue PSDs are eliminated using the digital multiplication of the signal and the reference. Analogue sine-wave generators are susceptible to amplitude drift: especially as a function of temperature. In contrast, the digital reference sine wave has a precise fixed amplitude. This avoids a major source of gain error common to analogue lock-ins. For applications that require high precision, short detection time, digital lock-in amplifiers are preferred to the analogue ones. Flow-cytometry is one such application.

To detect and identify particles in a microfluidic channel using the approach of AC impedance spectroscopy, the analogue input is a modulated signal that passes through a particle-containing fluid. It is applied as AC voltage at excitation frequency $f_0$ between detection electrodes normally positioned on both sides of a common microfluidic channel in the detection area of the channel. The voltage is applied to one of the two opposite electrodes and the signal is taken from the second electrode and sent to the lock-in amplifier. The signal is a complex function of the impedance of the cell, impedance of the elements of the cell, impedance of the fluid surrounding the cell, dielectric properties of the cell and its elements, dielectric properties of the fluid carrying the cell, the shape of the electrodes, size of the cell compared to the size of the electrodes, position of the sell with respect to the electrodes and orientation of the cell with respect to the electrodes. Dielectric properties of the cell and the fluid as well as impedance of the cell and the fluid are complex functions with real and imaginary components. All these also depend of the excitation frequency $f_0$.

Complete analytical description of the system in terms of the first principles is too complex and is far beyond the scope of this document. However, the complete analytical description is also unnecessary for the detection and identification of cells. The detection of cells is based on changes in the signal send to the lock-in amplifier resulting from the individual passing cells. The identification of the single particles (cells) is based on a principle: identical particles (cells) positioned in the same way with respect to the electrodes, will produce identical signals.

The signal induced is normally very small and it comes against significant noise background and therefore lock-in detection is used to demodulate the signal, extract it from the noise and measure it. It is important to note that the signal from the particles (cells) comes at frequency $f_0$, the same as the excitation frequency. After multiplying the modulated signal by a sinusoid with frequency $f_1$, we obtain a sum of sinusoids with different frequencies, i.e. a combination of modulation frequency and reference frequency, as described above. By applying the correct type of filter is possible to isolate the sinusoid with the frequency $f_0$ we are interested in. After the demodulation, the signal must be sent via low-pass filter. There are number of options for the low-pass filter such as: FIR (Finite length Impulse Response) filters and IIR (Infinite length Impulse Response) filters. IIR filters contain feedback, whereas FIR filters do not. As a result the nth output sample, y[n], of an FIR filter is a function of only a finite number of samples of input sequence, whereas the nth sample of an IIR filter may depend upon an infinite number of samples. FIR filters are mostly implemented directly using a relation similar to the convolution sum, although they can also be implemented using discrete Fourier transforms (DFTs).

Paper [T.Sun and H. Morgan, Single-cell microfluidic impedance cytometry: a review, Microfluid. Nanofluid, 2010, 8,423-443] describes several methods where cells flow between miniature electrodes which have an AC field applied across them. As the cell passes between the electrodes, the current path is disturbed and the change in current gives a change in the impedance signal associated with a single cell. Usually, impedance measurements at the frequency of (1-5 MHz) give information on the cell membrane capacitance whilst much higher frequencies (>10 MHz) probe the internal properties of the cell. Two or more frequencies can be applied simultaneously to differentiate different types of cells.

The inventors have also investigated impedance flow spectroscopy method of cell detection where two pairs of electrodes are used similar to configuration described in ["Microfluidic impedance cytometer for platelet analysis", Mikael Evander et.al, Lab on a chip, volume 13, 2013], each pair having an excitation and measurement electrodes. An AC voltage at radio frequency from 100 KHz -100 MHz is applied to an excitation electrodes and an electrical current is measured by the measurement electrodes. The electrical current being measured is then amplified and converted into an output voltage. The output signal is then demodulated to remove excitation frequency and to recover impedance magnitude and phase. As a cell passes through the pair of excitation and measurement electrodes, impedance magnitude and phase change, thus recording the information about the cell properties. Additional pair of electrodes ensures measurement is differential thus eliminating parasitic electromagnetic noise. Typically, the impedance measurements are taken at low frequency: 100KHZ-2MHz to acquire information about the cell size and at high frequency 2-100MHz to acquire information about the cytoplasm and internal properties of the cell.

Impedance flow cytometry can readily detect a cell, and the original technique was developed by Coulter for this. When it comes to more challenging task of identifying the sub-populations of cells within the sample fluid, the performance of the impedance cytometry is much less convincing due to large spread in the data points corresponding to each cell. Hydrodynamic focusing is particularly important for the detection of cells and particles on a chip utilizing impedance measurements. Indeed, for identification of cells (or particles) it is necessary to arrange these in such a flow that they pass in front of the detection system one by one. This "one cell-by-one cell" principle is fundamental for the successful cell identification: one needs to avoid the situation of multiple cells passing through the detection system at once as it could prevent the identification. It is common to use hydrodynamic focusing to achieve this. Hydrodynamic focusing is based on injection of the sample fluid into the laminar flow of sheath fluid. The two flows then merge into to a single channel, usually of a reduced cross-section. This reduces the cross-sections of both, the sheath fluid part of the flow and also the sample liquid flow, and thus achieves the desired reduction in the cross-section of the sample fluid flow. To control the cross-section of the sample fluid, one could change the flow rates of the sample fluid and sheath fluid. In relation to the electrical impedance-based cytometry, hydrodynamic focussing reduces the width of the conductive sample stream to the appropriate size of the cells, increasing the percentage resistance change in the conductive path when a cell passes by.

In recent years there is increasing body of work on the use of hydrodynamic focusing in microfluidic chips and microchannels. For example, the Japanese patent laid-open No 2003-107099 discloses a "fractionation microchip having a channel for introducing a particulate-containing solution, and a sheath flow forming channel arranged on at least one lateral side of the of the introducing channel. The particulate fractionation microchip disclosed in Patent 2003-107099, is so designed that fluid laminar flows are formed by a "trifurcated channel" having a channel for introducing a particulate-containing solution and two sheath flow-forming channels. In essence this is a 2D hydrodynamic focussing on a chip. Similar approach is described in [R. Rodriguez-Trujillo, C. Mills, J. Samitier, G. Gomila, Microfluid. Nanofluid, 3 171 (2007)] and [P.Walsh, E. Walsh, M. Davies, Int. J. Heat Fluid Flow 28 44 (2007)].

There are also solutions for integration of 3-D hydrodynamic focussing with a conventional type microfluidic chip described in ["Three-dimensional hydrodynamic focussing in a microfluidic Coulter counter", R. Scott, P. Sethu, C.K. Harnett, Rev. Sci. Instruments 79 046104 (2008)]. A similar approach is described in ["Universally applicable three-dimensional hydrodynamic microfluidic flow focussing" Yu-Jui Chiu, S.H. Cho, Z. Mei, V. Lien, T.F. Wu, Y.H. Lo, Lab Chip 2013 13 1803]. That study deals with three-dimensional hydrodynamic focusing where the sample channel and the two sheath channels having a greater height than the sample channel, join at the junction before the main channel which has the same height as the sheath channel. The merging of channels of different heights produces flow confinement both in the lateral and transverse directions, resulting in 3D focused flow. In the patent WO 2008/125081 A1 Theisen Janko et al provides the method for focusing fluid in microfluidic channel structure and the implementation of such a microfluidic structure to achieve hydrodynamic focusing of fluid. In the patent US 2009/0283148 A1, Shinoda et. al. [US 2009/0283148 A1, "Microchip and channel structure for the same", Masataka Shinoda, May 4 2009] teaches the method of three dimensional hydrodynamic focusing where the microtube is inserted into the microchip to providing the sample flow.

Despite these efforts, integration of 3D hydrodynamic focusing with microfluidic chip is not simple and the performance of such on-chip 3D focusing has limited capability. To reduce the variation of the impedance flow cytometry measurements, it is desirable to be able to direct the sample flow through a well-defined point in between the electrodes, e.g. the centre of the channel. This reduces the spread in the data points from a single population of cells of type of particles in the flow. It may also be desirable to align all the cells (particles) in the same way with respect to the direction of the electric field created by the electrodes. Cells often do not have an overall spherical shape but are rather elongated, ellipsoidal or discoid in shape. The signal from the cell in electrical impedance cytometry device depends on the orientation of the elongated axis of the cell with respect to the electrodes.

Having reviewed the methods and technologies for identification of particles/cell, especially those based on AC impedance spectroscopy, we are now moving to the issue of particles/cells separation. Once the particles/cell are identified it is often necessary to separate different sub-sets of particles/cell into different streams or different containers. One method is based on ejecting the flow of liquid containing particles/cell via a termination at the end of the microfluidic channel so that it breaks into minute droplets, one drop containing one particle/cell. This can be done by applying high voltage to the end of the channel. For this, a high-voltage generator is connected to the end of the microfluidic channel. This is also sometimes referred to in the industry as electrospray. The shape of the microfluidic channel termination is essential for the process of splitting the continuous jet emerging from the channel into a train of droplets. Therefore, commonly a nozzle is attached at the end of the channel to introduce control and reproducibility into the process of splitting the continuous jet into a train of droplets. The droplets are charged by the very same high voltage generator connected to the microfluidic channel. Then the droplets pass through an area of an electric field that is generated by control electrodes that are parallel to the pathways of the passing droplets. The electric field is perpendicular to the pathways of the passing droplets. The droplet trajectories ejected from the nozzle into the space in between the control electrodes, can be altered by applying a control voltage to the control electrodes. The control voltage is applied in the form of a pulse and this needs to be coordinated with the output of the device measuring and identifying the cells (e.g. fluorescence detector) so that particles/cells of the desired sub-set of particles cells move into a different trajectory and arrive to a required destination container. There are many embodiments of technology utilising this principle with various methods of forming drops/aerosols containing particles/cells. For further details one can refer to [D.Parks, B. Sahaf, O.Perez, M. Roederer, L.A. Herzenberg, Clinical Chemistry, (2002) 48: 1819-1827; W.A. Bonner, H.R. Hulett, R.G. Sweet, L.A. Herzenberg, Review of Scientific Instruments, (1972) 43: 404-409 ].

There is one disadvantage of this technology. It is convenient to have separation of particles/cells achieved in-situ, in the very same microfluidic chip, as opposed to letting the cells leave the microfluidic device into the ambient and perform separation in the form of droplets in the ambient.

US patent application 2009/0283148 A1 to M. Shinoda and T. Takashimizu attempts to address this issue. The particles are packed into droplets travelling through a microfluidic channel. Along the channel, there are positioned two electrodes generating electrostatic field applying a Coulomb force acting on the droplets.

There are a number of publications and inventions related to the use of electrophoretic, dielectrophoretic or electroosmotic forces for separation of cells. We shall therefore explain the terms electrophoresis, dielectrophoresis and electroosmosis and forces resulting from these. In electrophoresis the particles/cells are charged by their interaction with the surrounding fluid and the charged particles move along or against the direction of electrostatic field. The force is proportional to the charge of the particle/cell. Examples of systems utilising electrophoretic forces can be found in [J. Voldman, Annual Review of Biomedical Engineering, (2006), 8: 425-454. Pub Med 16038291;

K. Takahashi, A. Hattori, I. Suzuki, T. Ichiki, K. Yasuda, Journal of Nanobiotechnology, 2004: 2. Pub Med: 15056390]. Publication [F. Guo, X-H Ji, K. Liu, R-X. He, L-B. Zhao, Z-X Guo, W. Liu, S-S Guo, X-Z. Zhao Applied Physics Letters 2010; 96; 193701] describes the extension of the electrophoresis technique to sorting water-in-oil droplets under continuous flow.

In contrast to electrophoresis, where cells move in a uniform electric field due to their surface charge, dielectrophoresis refers to the movement of particles (cells) in a non-uniform electric field due to their polarizability. For movement in response to a dielectrophoretic force, particles (cells) do not need to possess a surface charge. The surface charges are produced by the field by disproportionation (pulling) the opposite charges to different parts of the cell. Once exposed to a gradient field, cells migrate either towards or away from the region of strongest field intensity depending on the electrical permeability (permittivity) of the cell and the fluid. Cells with a higher permeability than the fluid are attracted toward the field maxima, which is known as positive dielectrophoresis. The opposite is true for negative dielectrophoresis. The magnitude of the dielectrophoretic force is dependent on the size and properties of the cell, fluid, and the parameters of the electric field, which is useful for sorting cells by size and dielectric properties. Review of the phenomenon for the separation of particles/cells can be found in [J. Voldman, Annual Review of Biomedical Engineering, (2006), 8: 425-454. Pub Med 16038291]. We stress that the dielectrophoresis is based on gradient-, i.e. non uniform field. Usually papers described using AC gradient field, i.e. AC field with amplitude that varies strongly in space. The use of AC field is preferable as DC field may be screened by a conducting liquid surrounding the cells or by the cells themselves.

Dielectrophoresis force was used by a number of authors for the separation of cells and other microscopic objects. Review [M.P. Hughes, Strategies for dielectrophoretic separation in laboratory on chip format, Electrophoresis 2002, 23, 2569-2582] describes strategies for the separation of bioparticles such as cells, viruses and proteins in miniaturised laboratory-on-a-chip format. The separation principle in based on difference in polarisation of particles. The review describes various electrodes such as stepped electrodes, "Christmas tree" electrodes, spiral electrodes creating controlled gradient (non-uniform) electric field. Publication [B.H. Lapizco-Encinas, B.A. Simmons, E.B. Cummings, Y. Fintchenko, Dielectrophoretic concentration and separation of live and dead bacteria in an array of insulators, Anal Chem 2004, 76, 1571-1579] describes the use of dielectrophoresis for separation of dead and alive cells held within a body of an insulator. The paper [N. Lewpiriyawong, K. Kandaswamy, C. Yang, V. Ivanov, R. Stocker, Microfluidic characterization and continuous separation of cells and particles using conducting poly(dimethyl siloxane) electrode induced alternating current-dielectrophoresis, Anal. Chem. 2011, 83, 9579-9585] describes separation of live yeast cells from the dead ones in a microfluidic channel under the influence of the dielectrophoretic forces in gradient electric field along the channel. The principle is based on having a different value of the dielectrophoretic force acting on live and dead cells. Other publications [H. Shafiee, M.B. Sano, E.A. Henslee, J.L. Caldwell, R.V. Davalos, Selective isolation of live/dead cells using contactless dielectrophoresis (cDEP), Lab Chip, 2010, 10, 438-445] and [H. Li, R. Bashir Dielectrophoretic separation and manipulation of live and heat treated cell of Listeria on microfabricated devices with integrated electrodes, Sensors and Actuators B 86 (2002) 215-221] describe a similar approach. US patent 7,294,249 to S. Gawad, M. Wuethrich, P. Renaud, describes microfluidic component and method for sorting particles in a fluid utilising such a dielectrophoretic force. For this there are electrodes made protruding over the micorfludic channel generating gradient electric field applying dielectrophoretic force on the particle as they flow through the channel.

A number of publications use the approach described earlier in relation to the electrophoresis, i.e. encapsulating single cells into emulsified droplets and sorting he droplets using dielectrophoretic force [J.J. Agresti, E. Antipov, A.R. Abate, K. Ahn, A.C. Rowat, J.C. Baret, M. Marquez, A.M. Klibanov, A.D. Griffitths, D.A. Weitz, Proceedings of the National Academy of Sciences of the United States of America, 2010; 107: 4004-4009; L. Mazutis, J. Gilbert, W.L. Ung, D.A. Weitz, A.D. Griffiths, J.A. Heyman, Nature Protocols, 2013, 8: 870-891].

Electroosmotic sorting of two different types of E.coli cells was demonstrated in [A.Y. Fu, C. Spence, A. Scherer, F.H. Arnold, S.R. Quake, Nature Biotechnology, 1999; 17: 1109-1111]. In this work fluorescence was used to identify the cells and the difference was that one type of E.coli expressed a particular protein while the other one did not. The separation was demonstrated in the format of two microchannels forming a T-junction. There were three terminals for making electric contacts at the three distal ends of the microchannels forming the T-junction. A DC voltage was applied between the three terminals forming potential difference between the terminals and therefore resulting in electroosmotic pumping. High separation speeds are described, but passing electrical current along the full length of the microfluidic channel is not optimal for living cells and limits the application of the methodology. In addition, high voltages are required due to the length of the electrical field which would lead to bubble formation along the separation electrodes. It is an object of the invention to overcome at least one of the above-referenced problems. Similar approach was demonstrated in [P.S. Dittrich, P. Schwille, Analytical Chemistry, 2003; 75: 5767-5774] for separation of Escherichia Coli, again based on fluorescence detection. Silicon microfluidic chip was used and the connection of the voltages to drive electroosmosis was similar to the one in the publication cited previously. The voltage was applied along the channels and the switching was demonstrated in the DC mode as opposed to cell sorting at high pace.

All these publications and inventions describing the use of electrophoretic or dielectrophoretic force for the separation of particles/cell pursue a different paradigm to the one described in this invention. Their approach is based on using the force that selectively interacts with one sub-set of particles/cell but not with the others, e.g. it has significant interaction with live cells but little or no interaction with dead cells. Therefore the separation is done by the selective mechanism of effect of the stimulus on cells: some of them are affected by the stimulus while others are not.

We would like to develop a separation method where the interaction stimulus, once it is switched on, acts on the entire flow of particles/cells including ALL the sub-sets of particles/cells in the flow not just one sub-set. Furthermore, we would like to be able to switch this interaction on and off as required to guide the cells into the correct destination locations at high repletion rate. The rate of activating/deactivating the interaction should be as high as 100-10000 interactions per second or even higher. In this sense our requirements are similar to the ones delivered by the electrostatic separation of cells packed in droplets as described above but with the added restriction that the separation is done in the on-chip format.

There are some other methods are based on applying a force or pressure pulse to the flow, or fragmenting the flow into droplets and applying a force to defect the droplets. These methods are significantly outside the scope of the present invention. Therefore we will only give a brief review of these. There are methods based on acoustic streaming forces generated from non-standing surface acoustic waves to redirect fluid flow from one outlet to an adjacent outlet. An example of these can be found in [T.Franke, S. Braunmuller, L. Schmid, A. Wixforth, D.A. Weitz, Lab Chip. 2010; 10: 789-794]. There are methods based on separation of cells based on standing acoustic waves [S.C. Lin, X. Mao, T.J. Huang, Lab. Chip. 2011; 11: 1280-1285; S.C. Lin, X. Mao, T.J. Huang, Lab. Chip. 2012; 12: 2766-2770].

There are methods based on optical manipulation [A. Jonas, P. Zemanek, Electrophoresis, 2008, 29: 4813-4851]. There are methods based on generating magnetic force. We will not review these as they are not related to the invention and are limited to magnetic particles. There are methods based on generating a pressure pulse in the channel by a mechanical actuator, e.g. by a piezoactuator.

Summary of the Invention

The present invention addresses the problems of the device of Fu et al and provides an on-chip microfluidic cell sorter that employs electroosmotic separation of cells without damage to the cells or the need for high voltages known to produce bubbles at the electrodes. Another problem with the current approach is that cells are subjected to electric current all along the channel, i.e. the cells are subjected to current for extended length of time, not just to a short pulse of current. The extensive exposure to electric current affects the cells condition, e.g. viability of sperm cells. The invention employs separation electrodes in the separation zone of the microfluidic channels to generate an electric current/field that is limited to the separation zone. Unlike Fu et al, which teach an electrical field/current that extends along the whole of the microfluidic channel, the present invention employs separation electrodes that are disposed in the sidewall of the microfluidic channel in the separation zone, and thus produces an electrical field/current that is generally orthogonal or tangential to the direction of flow of the fluid in the microfluidic cannel. Therefore, each cell is subject only to a single short pulse of current. For selection of a single sub-set of cells out of a set of cells one could even entirely avoid the need for current pulse as will be explained below.

According to a first aspect of the present invention, there is provided an apparatus for separation of a particulates in a fluid into subsets of particulates, the apparatus comprising a microfluidic chip comprising:a microfluidic channel having a fluid inlet for receipt of a stream of particulate containing fluid, a detection zone disposed in the microfluidic channel and comprising a sensor configured to detect changes in the microfluidic channel corresponding to anisotropic particles passing the sensor, and a separation zone distal of the detection zone in which the microfluidic channel divides into at least two secondary microfluidic channels. The separation zone typically comprising a pair of separation electrodes including a first separation electrode disposed in electrical contact with an interior of the microfluidic channel and a second separation electrode disposed in electrical contact with an interior of the microfluidic channel or one of the secondary microfluidic channels. The pair of separation electrodes are typically configured during use to pass a pulse of current through the microfluidic channel.

In one embodiment, the apparatus comprises a voltage/current generator electrically connected to at least one or more of separation electrodes and configured to supply a voltage/current pulse to these/this separation electrodes.

In one embodiment, the apparatus comprises a voltage/current generator electrically connected to at least one or more separation electrodes and configured to supply a different voltage/current pulse to each of the pair of separation electrodes.

In one embodiment, the voltage/current generator is configured to apply a voltage pulse to the separation electrode in the range of 0.01-50 V of either positive or negative polarity.

In one embodiment, the voltage/current generator is configured to apply a voltage pulse to the separation electrode with the duration in the range of 1 microsecond to 50 milliseconds.

In one embodiment, the voltage/current generator is electrically connected to one of the pair of separation electrodes and the other of the pair of electrodes is connected to a fixed potential.

In one embodiment, the apparatus includes a processor operatively connected to the sensor and the or each voltage/current generator and configured to receive signals from the sensor in the detection zone and actuate the or each voltage/current generator in response to signals received from the sensor.

In one embodiment, both of the separation electrodes are disposed in electrical contact with the microfluidic channel.

In one embodiment, the separation electrodes are disposed on opposite sides the microfluidic channel.

In one embodiment, the separation electrodes are longitudinally aligned so that during use the electrical field generated by the electrodes is substantially orthogonal to a longitudinal axis of the microfluidic channel.

In one embodiment, the separation electrodes are longitudinally offset so that during use the electrical field generated by the electrodes is tangential to a longitudinal axis of the microfluidic channel.

In one embodiment, one of the pair of separation electrodes is disposed in electrical contact with the microfluidic channel and another of the pair of separation electrodes is disposed in electrical contact with one of the secondary microfluidic channels.

In one embodiment, the apparatus includes two pairs of separation electrodes.

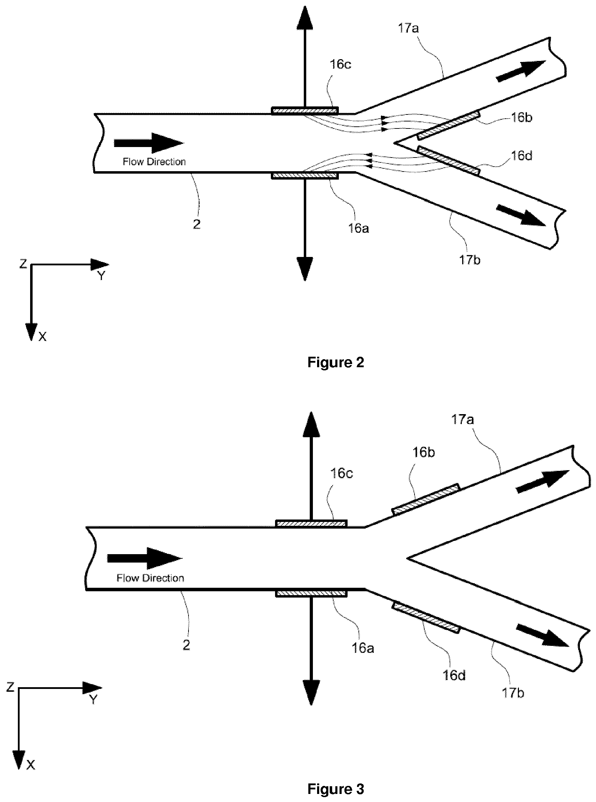

In one embodiment, the apparatus includes two electrodes in the microfluidic channel and an electrode in each of the two secondary microfluidic channels. In one embodiment, the electrodes in each of the two secondary microfluidic channels are disposed on an inner sidewall of the secondary microfluidic channels (i.e. Fig. 2). In one embodiment, the electrodes in each of the two secondary microfluidic channels are disposed on an outer sidewall of the secondary microfluidic channels (i.e. Fig. 3).

In one embodiment, the sensor in the detection zone is configured to measure AC impedance change between a set of detection electrodes positioned in the microfluidic channel corresponding to particulates obstructing the electric path between the detection electrodes.

In one embodiment, the set of detection electrodes comprises two pairs of detection electrodes.

In one embodiment, the or each set of detection electrodes comprises an energising electrode and a signal electrode electrically coupled to a lock-in amplifier.

In one embodiment, the sensor is configured to make optical measurements of the light scattered by the particulates passing along the detection zone of the microfluidic channel.

In one embodiment, the sensor is configured to make optical measurements of the fluorescence signal from the particulates passing along the common microfluidic channel.

In one embodiment, a cross-section of microfluidic channel perpendicular to the flow direction has a width of 20-500 $\mu$m and a height of 20-500 $\mu$m.

In one embodiment, the linear velocity of the fluid in the middle of the common microfluidic channel is 10 mm/sec.

In one embodiment, the apparatus includes a hydrodynamic focussing mechanism configured to hydrodynamically focus the stream of particulate containing fluid to provide a focussed sample fluid within a guidance/sheath fluid.

In one embodiment, the apparatus includes an on-chip hydrodynamic focussing mechanism comprising a sample channel and a guidance fluid channel that merge at an oblique angle to form the microfluidic channel.

In one embodiment, the apparatus is further equipped with means for positioning of particles/cells within the cross-section of the microfluidic channel.

In one embodiment, the cross sectional area of the secondary microfluidic channels is greater than the cross sectional area of the microfluidic channel.

In another aspect, the invention provides a method to separate particulates in a particulate containing fluid, which method employs an apparatus according to the invention. The method suitably comprises the steps of passing a particulate containing fluid along the microfluidic channel, employing the sensor to detect a change in the microfluidic channel corresponding to a particulate passing the sensor in the detection zone, and actuation of the separating electrodes in the separation zone in response to the change detected in the microfluidic channel to pass a pulse of current through the microfluidic channel and deflect the particulate into one of the secondary microfluidic channels.

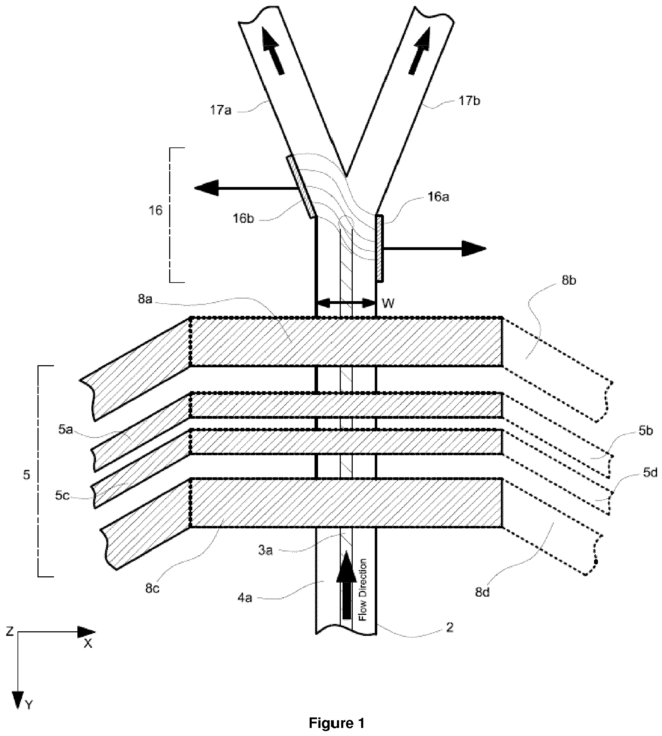

The method of the invention may be employed to separate a particulate containing fluid into at least first and second subpopulations of cells (for example X and Y subpopulations of semen cells). The separation electrodes may be configured to pass a single pulse of current through the separation zone of the microfluidic channel (i.e. a pulse corresponding to one of the subpopulations), which separates one of the subpopulations of cells. In this embodiment, the microfluidic channel can suitably bifurcate to provide a side secondary channel configured to receive deflected cells (i.e. one subpopulation), and a main secondary channel configured to receive non-deflected cells (i.e. the other subpopulation or sub-populations). Thus, the main secondary channel which is nearly co-linear with the microfluidic channel, and a side secondary channel which diverts away from the main channel. In another embodiment, the separation electrodes may be configured to pass more than one pulse of current through the separation zone of the microfluidic channel in a sequential manner corresponding to the signals received in the detection zone (i.e. a first pulse corresponding to one of the subpopulations, and a second pulse corresponding to another of the subpopulations). In this embodiment, the secondary channel may take the bifurcated form shown in Figs 1-5.

In one embodiment, the invention is for separating a first and second subpopulations of particulates from the particulate containing fluid, including the steps of:

detecting a first change in the microfluidic channel corresponding to a particulate of the first subpopulation passing the sensor in the detection zone;
actuation of the separating electrodes in the separation zone in response to the first change detected in the microfluidic channel to pass a first pulse of current through the microfluidic channel and deflect the particulate of the first subpopulation into a first of the secondary microfluidic channels;
detecting a second change in the microfluidic channel corresponding to a particulate of the second subpopulation passing the sensor in the detection zone; and actuation of the separating electrodes in the separation zone in response to the second change detected in the microfluidic channel to pass a second pulse of current through the microfluidic channel and deflect the particulate of the second subpopulation into a second of the secondary microfluidic channels.

In one embodiment, the invention is for separating a first and second subpopulation of particulates from the particulate containing fluid, and in which the secondary microfluidic channels include a side secondary channel configured to receive deflected particulates pass and a main secondary channel configured to receive un-deflected particulates, the method including the steps of:

detecting a first change in the microfluidic channel corresponding to a particulate of the first subpopulation passing the sensor in the detection zone;

actuation of the separating electrodes in the separation zone in response to the first change detected in the microfluidic channel to pass a first pulse of current through the microfluidic channel and deflect the particulate of the first subpopulation into the side secondary microfluidic channels; and
allowing the un-deflected second subpopulation of cells pass into the main secondary channel.

In one embodiment, the particulates are anisotropic particulates.
In one embodiment, the particulates are cells, and in which the method separates the cells according to a phenotype (for example, cell type, cell status (dead or alive), cell sex, cell health etc).
In one embodiment, the particulates are semen cells, and in which the method separates the cells according to a sex (i.e. into X and Y populations).
In one embodiment, a first voltage is applied to the first separation electrode and a second voltage is applied to the second separation electrode.
In one embodiment, a first voltage is applied to one of the separation electrode pair and the other of the separation electrode pair is connected to a fixed potential.
In one embodiment, a first voltage and second voltage are each, independently, positive or negative polarity.
In one embodiment the voltage pulses applied to the separation electrode/electrodes are of a substantially non-rectangular shape such as bell-shaped or pulses with smeared limiting fronts of the pulses signifying the start and the end of the pulse.
In one embodiment the separation zone is separated from the detection zone by means of shielding electrodes connected to a fixed potential or ground potential.
In one embodiment, the apparatus comprises two pairs of separating electrodes including two electrodes in the microfluidic channel and an electrode in each of the two secondary microfluidic channels, wherein the two electrodes in the microfluidic channel are connected to a fixed potential and one of the electrodes in the secondary microfluidic channel is energised with a positive voltage and one of the electrodes in the secondary microfluidic channel is energised with a negative voltage.
In one embodiment, the apparatus comprises two pairs of detection electrodes, and in which the method includes a step of determining the speed of particulate fluid in the microfluidic channel by detecting the time it takes a particulate to pass from a first pair of detection electrodes to a second pair of detection electrode.
In one embodiment, the determined speed of the particulate containing fluid is correlated with a distance between the first pair of detection electrodes and the separation electrodes to calculate a time delay between (a) detecting a change in the microfluidic channel associated with a particulate passing the detection electrode and (b) actuation of the separating electrodes to deflect the particulate into one of the secondary microfluidic channels.
Other aspects and preferred embodiments of the invention are defined and described in the other claims set out below.

Brief Description of the Figures

**Fig. 1** is a plan view of a microfluidic chip forming part of an apparatus of the invention showing the microfluidic channel, detection zone and separation zone having one pair of separation electrodes that span the separation zone in a longitudinal and transverse direction.

*Fig. 2* is a plan view of a microfluidic chip forming part of an apparatus according to an alternative embodiment of the invention showing the microfluidic channel and separation zone having two pairs of separation electrodes.

*Fig. 3* is a plan view of a microfluidic chip forming part of an apparatus according to an alternative embodiment of the invention showing the microfluidic channel and separation zone having two pairs of separation electrodes.

*Fig. 4* is a plan view of a microfluidic chip forming part of an apparatus according to an alternative embodiment of the invention showing the microfluidic channel and separation zone having one pair of separation electrodes that span the separation zone in a longitudinal direction.

*Fig. 5* is a plan view of a microfluidic chip forming part of an apparatus according to an alternative embodiment of the invention showing the microfluidic channel and separation zone having one pair of separation electrodes that are disposed proximal to the bifurcation of the microfluidic channel and in use generate an electrical field that is orthogonal to a direction of flow of sample fluid in the microfluidic channel.

*Fig. 6* is a plan view of an apparatus of the invention showing the detection zone with lock-in amplifier, separation zone voltage /current generator.

*Fig. 7* is a sectional view taken along the line A-A of Fig. 6 showing the detection electrode having an energising electrode below the microfluidic channel and a signal electrode disposed above the microfluidic channel.

*Fig. 8* is graph of voltage V vs time of the output signal obtained from the comparator of Fig. 6.

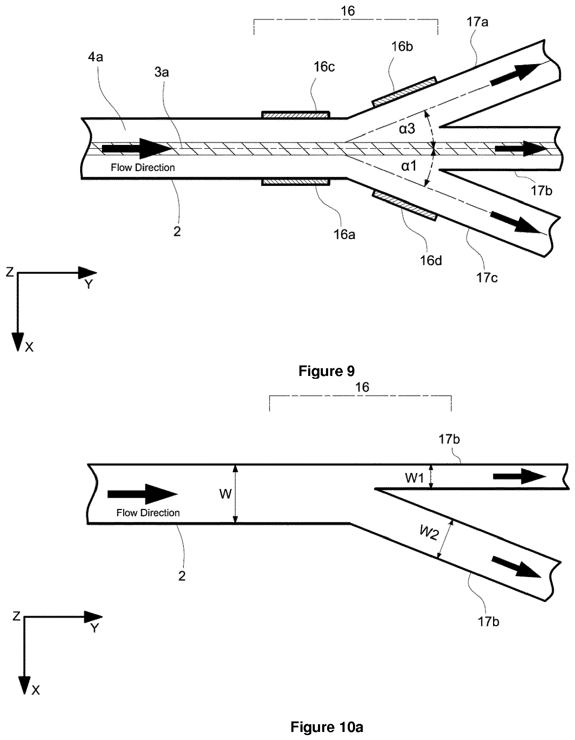

*Fig. 9* is a plan view of a microfluidic chip forming part of an apparatus according to an alternative embodiment of the invention showing the microfluidic channel which bifurcates into three channels and separation zone having two pairs of separation electrodes.

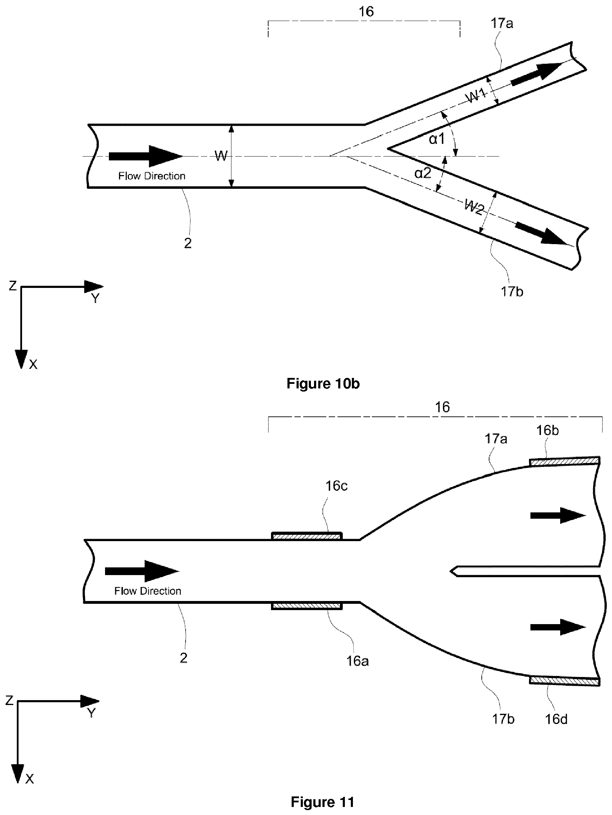

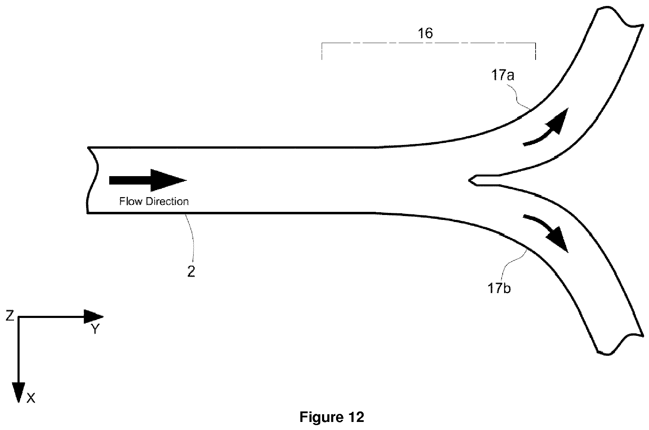

**Figs 10a,10b, 11 and 12** are illustrations of microfluidic channels having secondary channels of different dimensions and architecture.

## Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

## Definitions and general preferences

Where used herein and unless specifically indicated otherwise, the following terms are intended to have the following meanings in addition to any broader (or narrower) meanings the terms might enjoy in the art:

This invention deals with instruments for counting, identification and separation of particles in particulate-containing fluids such as e.g. flow cytometry or particle flow analyser. For many common envisaged applications in this invention, the particles in the fluid are cells, and particulate containing fluid could be a suspension of mammalian cells, semen, blood cells, viruses, bacteria or indeed any other type of isolated particles. However, the technology could also be used with numerous other types of particles: organic, inorganic, ceramic, composite, nanoparticles, etc., both solid, semi-solid or liquid (e.g. protein particles, fatty particles, blends of soft particles and their compositions, drops of one liquid in the stream of another one, etc). The solid particles could be particles of metals, oxides, nitrides, sulphides, polymer particles and particles of numerous other inorganic and organics materials, also mixed particles containing blends and composites of materials within individual particles and various nano- and micro-particles and clusters. We shall use the term "particle" or "cell" to cover any and all of these. We shall also use the same to describe clusters of particles/cells thus forming larger particle/cell. We shall call the liquid/fluid carrying particles in microfluidic channel as sample fluid, or particle containing fluid or particulate containing fluid.

The emphasis in the document is on technologies that can perform counting and identification of particles and/or cells in a microfluidic chip format. The said microfluidic chip has at least one microfluidic channel where such counting and identification takes place in the detection area (zone) of the channel. The preferred embodiment deals with the method of counting and/or identification of particles/cells based on AC electrical impedance spectroscopy. For each passing particle/cell, such counting and/or identification is normally done before the separation step.

The term "separation" of particles/cells is used to describe the process of physical separation of these, e.g. moving different subsets of the entire set towards different destination points. We will use the term "identification" of particles/cells to describe the process of attaining the information on the sub-set to which the particle/cell belongs to.

Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

"Particulate" as applied to a particulate containing fluid means a solid body in the fluid or a semi-solid, i.e. a body with properties different to that of the fluid. Examples include particles of metals, oxides, nitrides, sulphides, polymer particles, particles of inorganic or organic materials, particles of gel, also composite particles, and mixed particles, nanoparticles, microparticles, particulate complexes, cells, clusters of cells, bacteria, fungi, virus, clusters of viruses, particles of proteins. Likewise, "particulate containing fluid" means a fluid containing particulates. Examples include cell containing fluids, such as sperm containing fluid.

"Analysis" means determining a qualitative or quantitative characteristic of the particulates in the fluid, for example determining whether the particulates are a homogenous population or a heterogenous population, determining the amount or concentration of particulates, or differentiating or sorting the particulates based on differences. Thus, the term broadly covers analysis of the particulates (i.e. cells) qualitatively or quantitatively, or differentiation or sorting of the particulates based on detected impedance response differences.

"Cells" means any type of cell, including mammalian cells such as sperm, white blood cells, red blood cells, bone marrow cells, immune cells, epithelial cells, nerve cells, pulmonary cells, vascular cells, hepatic cells, kidney cells, skin cells, stem cells, or bacterial and fungal cells and hybridomas, yeast cells, cancerous cells. Generally, the particulate containing fluid contains at least two different types of particulates, for example different cell types, sperm of different sex, sub-populations of the same cell types, the same cell type having different phenotypes, dead and living cells, diseased and non-diseased cells, immature and mature cells of the same kind. The apparatus and methods of the invention may be employed to analyse and/or differentiate and/or separate these different types or phenotype of particulates/cells.

"Different phenotypes" as applied to cells means different populations of cells (i.e. hepatic cells and vascular cells), different sub-populations of the same cell type (i.e. different types of cartilage cells), different phenotypes of the same cell type (i.e. cell expressing different markers, diseased and healthy cells, transgenic and wild-type cells, stem cells at different stages of differentiation).

"X and Y population" as applied to sperm cells means male sperm and female sperm cells.

"Focussed stream of particulate containing fluid" means a fluid containing particulates in the form of a focussed beam of particulates positioned within a guidance stream. In one embodiment the particulates in the focussed beam are focussed into a single cell stream arrangement. In one embodiment, in which the particulates have an anisotropic shape, particulates in the focussed beam are aligned in the same direction.

"Microfluidic chip" means a chip having at least one microfluidic channel having a cross-sectional area of less than 1 mm$^2$ and a length of at least 1 mm. In one embodiment, the microfluidic chip has at least one microfluidic channel having a cross-sectional area of less than 0.25 mm$^2$. In one embodiment, the microfluidic chip has at least one microfluidic channel having a cross-sectional area of less than 0.01 mm$^2$. In one embodiment, the microfluidic chip has at least one microfluidic channel having a cross-sectional area of less than 0.0025 mm$^2$. In one embodiment, the microfluidic chip has a plurality of microfluidic channels, for example at least 2, 3, 4, 5, 6, 7, 8, 9 or 10 microfluidic channels. In one embodiment, the microfluidic chip has at least one microfluidic channel having a length of at least 1.5 mm. In one embodiment, the microfluidic chip has at least one microfluidic channel has a length of at least 2 mm. In one embodiment, the microfluidic chip has a length of at least 3 mm. In one embodiment, the microfluidic chip comprises a plurality of layers, for example at least 2, 3, 4, 5, 6, 7, 8, 9 or 10 layers.

"AC impedance changes" should be understood to mean changes in impedance detected between the detection electrodes. The changes may include changes in amplitude, phase, or amplitude and phase of the signal.

"In electrical communication with the microfluidic channel" as applied to the electrodes means that the electrodes are in direct contact with the fluids analysed in the microfluidic channel.

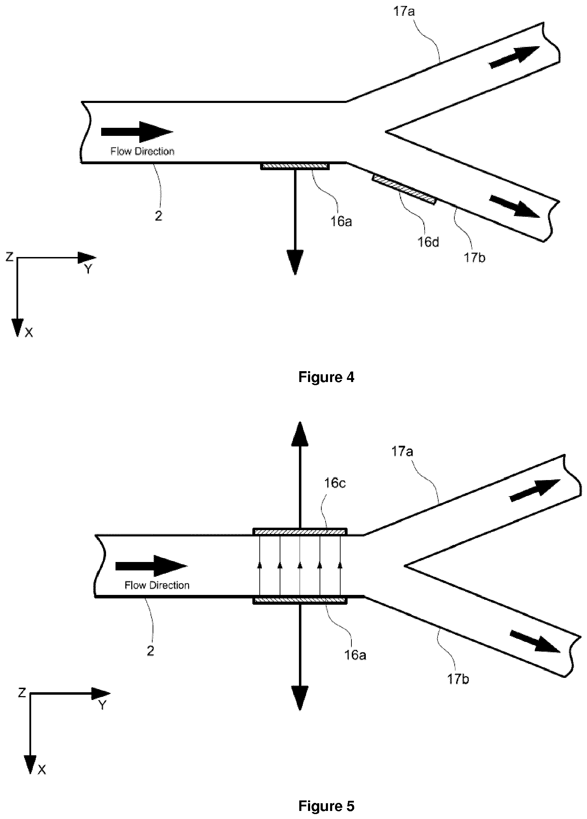

"Separation zone" is a part of the microfluidic chip, distal of the detection zone, where particulates in the fluid can be separated in accordance with the results of the characterization of the particulates in the detection zone. The separation zone comprises at least one pair of electrodes, optionally two pairs of electrodes. One or more Voltage/current generators are provided and electrically coupled to the separation electrodes. The or each voltage/current generator is configured to provide a pulse of current across the microfluidic channel. Generally, at least one separation electrode is disposed in the separation zone proximal (upstream) of the bifurcation (splitting) of the microfluidic channel, and at least one separation electrode is disposed either proximal (upstream) of the bifurcation (splitting) of the microfluidic channel or distal of the bifurcation (i.e. in one of the secondary channels. Thus, the separation electrodes may be disposed on opposite sides of the microfluidic channel proximal of the bifurcation (i.e. Fig. 5). The separation electrodes may be disposed proximally and distally of the bifurcation point (Fig. 1, 2, 3, 4). The separation electrodes may be disposed on the same side of the microfluidic channel and proximally and distally of the microfluidic channel (Figs 3, 4). The separation electrodes may be disposed on the opposite side of the microfluidic channel and proximally and distally of the microfluidic channel (Fig 2). One of the separation electrodes may be disposed on a side of the microfluidic channel proximal of the bifurcation and one may be disposed on a side |(inner side wall (Fig. 2) or outer sidewall (Fig. 3)) of a separation channel distal the bifurcation.

The term "anisotropic" refers to being not spherical in overall symmetry of particle's shape or its response to the stimulus used in the apparatus. In the simplest case, this refers to overall shape of the particle (cell). For example, if the particle is elongated, ellipsoidal, bar-shaped or disk-shaped, discoid, this is then described as anisotropic in contrast to a spherical shape particle that is being described as isotropic. However, the overall shape in its own right is insufficient to distinguish between anisotropic and isotropic particles (cells). For example, if a conducting rod (segment of wire) is embedded into an insulating sphere, this forms an anisotropic particle even if the overall shape of the particle is spherical, i.e. isotropic. The reason is that such a particle has different response to the Radio Frequency (RF) electromagnetic field depending on whether it is directed with the length of the rod along the field or perpendicular to the field. The main response to the RF field will be in this case from the metallic rod, this response will be highly anisotropic, the insulating spherical envelope will have little effect on the situation. The same applies to optical response: it will be different depending on the direction of the light incidence and the polarization with respect to the long axis of the rod, again the effect of the isotropic dielectric envelope on the optical response will not alter anisotropic response from the conducting rod. The same applies to the cells. The main contribution to RF signal response from a cell may not come from the exterior periphery of the cell but from its interior features. This depends on the structure of the cell and the RF frequency.

When referring to laminar flow regime, we shall imply the flow conditions that fall under the Stokes regime ($\sim$1<Re<$\sim$1000). Re is the Reynolds number defined as Re=$\rho$UH/$\mu$, where p, U and $\mu$, are the fluid density, the average velocity and dynamic viscosity respectively and H is the characteristic channel dimension. In some cases the effect of particle focusing may still be achieved when Reynolds number is below 1 and therefore the invention is not restricted to the situation of $\sim$1<Re<$\sim$1000. Generally the range of Re values at which the focusing is achieved, also depends on the difference between the density of the liquid and the density of the particles. The greater is the difference, e.g. the heavier are the particles compared to the liquid, the greater is the effective gravity force (difference between the gravity force and the buoyance force) pulling the particles down from the locations defined by the hydrodynamic forces. Therefore, the greater is the difference between the densities, the greater should be the force acting on the particles to achieve effective focusing of the particle's trajectories.

The term "separation of particulates in a fluid into subsets of particulates" should be understood to mean separating a type of particulate within a fluid. The fluid may contain just that particulate or more probably contains at least two subpopulations of particulates. The particulates may be cells, and may be separated into subpopulations based on phenotype, for example cell type, cell health (diseased, normal), viability (dead or alive), sex (X and Y subpopulations), level of differentiation (stem cells). The method employs an electric current to deflect specific cells, provided as a pulse. In one embodiment, the method of the invention employs at least two different current pulses, each configured to deflect a different subpopulation of particulate.

Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

Fig. 1 describes a section of an embodiment of a microfluidic chip comprising the common microfluidic channel 2, detection area 5 and the separation area 16. There can be other optional elements of the microfluidic chip such as area where a number of channels containing sample fluid 3a and the guidance (sheath) fluid 4a merge into the single common microfluidic channel 2. Again, this arrangement is optional. One may operate microfluidic chip without any sheath fluid 4a, for example by filling the entire cross-section of the microfluidic channel with sample fluid 3a alone. Such embodiment is also covered by the present invention. The optional area where multiple channels, sample channel and guidance microfluidic channel, merge into a common microfluidic channel, is not shown in Fig. 1 and most other figures of this document for brevity. This optional area is located upstream from the detection area 5. In this embodiment, the common microfluidic channel 2 has a rectangular cross-section with the dimensions in the following range: width w 20 to 3000 micrometres, more preferably 20 to 500 micrometres and the height h in the range of 10 to 3000 micrometres, more preferably 20 to 500 micrometres. The width w is the dimension along the X axis and it is shown in Fig.1 and the height h is the dimension along the Z-axis and it is not shown in Fig. 1. To simplify understanding of the invention it is best thinking in terms of a channel with rectangular cross-section. The cross-section of the common microfluidic channel 2 does not need to be square or rectangular, and channels with other cross-sections can also be deployed. The microfluidic chip could be also equipped with means for aligning anisotropic cells along a given direction. These means are not described here in further details. The chip may be equipped with a detection area where the particles/cell are identified. The detection area 5 is shown in Fig. 1 but it is not shown in some other figures. The detection area is normally located upstream from the separation area 16. The detection area 5 is equipped with detection electrodes 5a, 5b, 5c, 5d and optional shielding electrodes 8a, 8b, 8c, 8d to reduce the noise induced in the detection electrodes and improve signal detection. Such noise is manifested as voltage or current induced in the detection electrodes from various other electrodes of the microfluidic chip and various other sources of noise. The shielding electrodes 8a, 8b, 8c, 8d are connected to a fixed potential, e.g. a ground potential. The shielding electrodes are preferably in electric contact with the interior of the common microfluidic channel 2. One could also devise additional shielding electrodes on the microfluidic chip that are not in direct contact with the interior of the microfluidic channel. These mainly serve to shield the detection electrodes from the outside interference and induced voltage and noise, and are not shown in the figures of this document.

The detection electrodes normally have electric contact with the interior of the common channel but for some embodiments, especially operating at higher end of frequency range described in this document, the detection electrodes may also be electrically insulated from the interior of the common microfluidic channel. The detection electrodes 5a, 5b, 5c, 5d are connected to the detection circuit. Some detection electrodes are connected to AC voltage or current generators and others are connected to the AC voltage or current detectors. These are not shown in Fig. 1. We shall stress that some of the detection electrodes are connected to excitation AC voltage or current sources even though we attribute them to the set of detection electrodes; and other detection electrodes are connected to AC voltage or current meters/analysers. To differentiate between the two subsets of the detection electrodes, we shall call the detection electrodes connected to the AC voltage or current generator, the excitation electrodes; and the detection electrodes connected to the AC voltage current detector/meter, the signal electrodes. In the embodiment shown in Fig. 1, there are two excitation electrodes and two signal electrodes. They have the same dimensions and positioned just one under the other, one along the upper wall of the channel and the other one along the lower wall. The pair of electrodes which comprises one signal electrode and one excitation electrode is labelled a detection electrode.

The separation area 16 shown in Fig 1 has the common channel 2 splitting into two secondary channels. The secondary channels 17a and 17b are terminated with two sample collection reservoirs. This splitting of the common microfluidic channel into secondary channels is also called bifurcation. There are two separation electrodes 16a and 16b along the walls of the common microfluidic channel and secondary channels: one of these is located along the wall of the common channel and the other one is located along the walls of one of the secondary channels. The two separation electrodes are connected to two voltage/current generators. The voltage/current generators are capable of supplying voltage/current pulse with the duration in the range from 1 microsecond to some 100 milliseconds and the amplitude in the range of 1 mV to 100 V. The repetition rate of the pulses is variable. Under normal operation one pulse is supplied per one cell guided through the separation area. However, this does not need to be always the case. One could supply a chain of shorter pulses of duration say 10 microseconds instead of a single pulse of duration of 1 millisecond. The timing of the pulses is coordinated with signals from the detection area 5. The electrical connection from the control circuit of the detection area to the voltage/current generators of the separation area, is not shown in Fig. 1. How this is done, will be well understood by those skilled in the art of electronics. We shall discuss the issue of the timing of triggering of pulses supplied to the separation electrodes, below. Preferably the particles/cells are travelling in the middle of the common microfluidic channel 2 and in the absence of any voltages applied to the electrodes, they could equally well go into the secondary channel 17a or 17b. The flow of particles/cells in the common microfluidic channel is shown as sample fluid 3a. The sample fluid flow is positioned in the middle of the common microfluidic channel 2 and it is flanked by the sheath fluid 4a on both sides. This is known as hydrodynamic focussing, a phenomenon well known to those skilled in the art of microfluidics. As will be appreciated by those skilled in the art of microfluidics, the flow of sample fluid from the common microfluidic channel into the secondary channels is defined by the flow impedance of the secondary channels 17a and 17b from the point of bifurcation of the common channel into the secondary channels to the terminal point of the secondary channels (i.e. sample collection reservoirs). In the case of symmetric split and with the particles/cells positioned in the middle of the common microfluidic channel as shown in Fig. 1, equal splitting of the flow between the two secondary channels is achieved when the two secondary channels have equal fluidic impedance.

We teach in this patent application that such a symmetric situation will be altered if there is a voltage/current pulse applied between the electrodes. When the voltage is applied between the separation electrodes 16a and 16b, there is current tangential to the flow direction that will introduce non-equivalence into the entrances from the common microfluidic channel and the secondary channel 17a and the same for the secondary channel 17b. This can be considered as additional fluidic impedance or pressure source inserted between the common microfluidic channel and one of the secondary channels. The non-equivalence is results from the following key contributions. Firstly, there is electroosmotic pressure arising in the channel and this pressure will alter the difference between the distribution of flows of liquid into the secondary channels 17a and 17b. Secondly, there is electrophoretic force acting on the particles/cells. This force arises from the ionisation on the particles/cells that results from their interaction with the liquid in which they are immersed. The ionised particle/cell interacts with the electric field between the electrodes 16a and 16b and this produces the force acting on the particle/cell moving predominantly into the secondary channels 17a and 17b. Thirdly, there is dielectrophoretic force. This force results from the fact that the electric field between the electrodes 16a and 16b is not homogeneous and there is a region of gradient electric field. The dielectric permittivity of the particle/cells differs from that the liquid surrounding it. Therefore, there is force acting on the particle/cell that is proportional to the gradient of the electric field and the difference in the dielectric constants of the particle/cell and that of the liquid around it. Depending on the electric permittivity of the cell and the liquid carrying the particles/cells and also on the polarity of the voltage applied between the electrodes 16a and 16b, the particles/cells will be pulled towards the secondary channel 17a or 17b.

The quantitative effects of these three contributions to the force are difficult to describe theoretically. They depend on properties of the liquid, its pH, electric properties of the particle/cell, configuration of the electrodes.

The exact balance of the forces contributing to the overall force should preferably be determined experimentally and can be performed by a person skilled in the art. However, it is important that in any event, the introduction of the electric current between the electrodes as shown in Fig. 1 starts diverting cells into one of the two secondary channels, at the expense of the second secondary channel. Since all these contributions for the force described above scale up with value of the voltage applied between the separation electrodes, and in most cases they are proportional to the voltage, the net force also scales up with the voltage. The direction of the force is reversed once the polarity of the voltage is altered sending the particles/cells into the other secondary channel. Lines of the electric fields are also shown schematically.

The voltage/current pulse should be short enough and coordinate with the time of arrival of a specific cell that has been measured in the detection area 5, to the separation area 16.

Fig. 2 shows embodiment with four separation electrodes 16a, 16b, 16c and 16d. In this case one could apply voltage between the two sets of separation electrodes simultaneously of opposite polarities to enhance the force separating the particles/cells from the common microfluidic channel into one of the secondary channels. The two polarities of the voltage that favour the movement of particles/cells into the separation channels 17a and 17b are shown in Fig. 2. The polarities are shown schematically. As explained in relation to Fig. 1, the exact polarity depends on the properties of the particles/cells and those of the liquid around the particles/cells. Preferably the correct polarity needs to be established/confirmed experimentally. This is straightforward experimental procedure that needs to be carried out once for each new untested set of particles/cells. However, it is preferable in this embodiment that the potential difference from the electrode 16a to electrode 16d is opposite to the one from the electrode 16c to the electrode 16b. It is also important that by reversing the polarity as shown in Fig. 2, we can change the choice of the secondary channel receiving the particles/cells from the common microfluidic channel.

One example of sets of voltages applied to the four separation electrodes is now described: electrodes 16a and 16c connected to ground potential, electrode 16d connected to positive potential +V and electrode 16b is connected to negative potential-V. Electric fields produced by this set of voltages are shown schematically in Fig. 2. To reverse the selection of flow between the secondary channels 17a and 17b, one can maintain the potentials of separation electrodes 16a and 16c fixed, i.e. still connected to the ground potential and reverse the potentials applied to the electrodes 16b and 16d connecting electrode 16d to a negative potential -V and electrode 16b to a positive potential +V.

It is important to note that the voltages applied to the separation electrodes 16b and 16d do not need to be of the same value. For example, one could operate the separation device with the voltage of +10 V applied to the separation electrode 16b and voltage -5 V applied to the separation electrode 16d. In this case the separation force acting on the particles/cell will be smaller than in the case of identical voltage values: +10 V to the electrode 16b and -10 V to the electrode 16d. The electrodes 16a and 16c in Fig 2 are shown connected to the ground potential. This does not need to be the case. These could be connected to another potential as will be appreciated by those skilled in the art of mapping electric field generated by metallic electrodes. They could also be connected to different potentials.

Fig. 3 shows embodiment with four separation electrodes positioned in a somewhat different way around the channel bifurcation. These are connected to another set of potentials to achieve broadly similar effect of the forces to the one in Fig. 2. In this case the electrodes 16a and 16c are connected to the same potential V. To achieve transfer of particles/cells into one of the secondary channels, the electrode 16d is connected to the ground potential and the electrode 16b is connected to the same voltage V as the electrodes 16a and 16c. Those skilled in the art will appreciate that the voltage connected to the electrode 16b does not need to be the same as the one of electrodes 16a and 16c. This is given as a particularly simple specific example of energising the electrodes. To send the particles/cells into the alternative secondary channel, we reverse the connections of the electrodes: the electrode 16b is connected to the ground potential and the electrode 16d is connected to the voltage V.

Fig. 4 shows another embodiment of microfluidic chip, more specifically section of the chip at the point of bifurcation. Other sections of the microfluidic chip are not shown. This embodiment has just two separation electrodes 16a and 16d. The voltage of one of the electrodes is fixed. For example, separation electrode 16a could be connected to the ground potential with the second separation electrode 16d is connected to potential +V or - V for sending the particles/cells to one secondary channel or the other.

What is common is all these configurations is that all of them create electric field and current that is significantly non-collinear with the direction of the fluid flow in the common microfluidic channels in proximity of the point of bifurcation of the common microfluidic channel into a number of secondary channels.

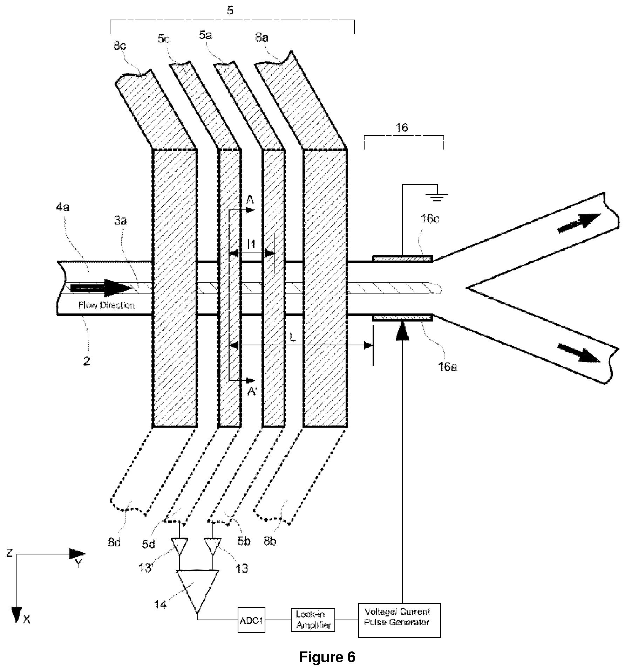

The voltage values applied to all these electrodes do not need to be constant in time. In a typical operation the voltages applied to the electrodes switch on and off or alter between the set values in order to achieve switching of the flow between different secondary channels. This is explained in further detail using Figs. 6, 7, 8.

Fig. 5 shows another embodiment having two separation electrodes 16a and 16c upstream of and in close proximity to the point of bifurcation. The current between the separation electrodes is perpendicular to the flow direction in the common microfluidic channel. This is shown schematically using lines of the electric field. To displace the particles/cells for subsequent migration into the selected secondary channel, one of the separation electrodes needs to be connected to a positive potential with respect to the other one and vice versa. The direction of the electric field in Fig. 5 is given for the case when the top electrode has higher potential than the bottom electrode.

The voltage applied to the electrodes can be switched on and off on demand. In most preferred embodiments the voltage to the electrodes is supplied in the form of pulses of fixed shape and amplitude: one pulse or one fixed train of pulses is supplied to direct a single particle/cell. In the typical embodiment, the generator/generators supplying the voltages to electrodes are controlled from the output of the circuit detecting the particles/cells. This is shown schematically in Fig. 6. The microfluidic chip shown in Fig. 6 has detection area 5 and separation area 16. The separation area 16 is similar to the one described in relation to Fig. 5 and it is located downstream of the detection area 5. There are two pairs of detection electrodes shown in the detection area. Each of these detection electrodes is composed of one excitation electrode and one signal electrode. The two excitation electrodes and the two signal electrodes in each pair of detection electrodes are positioned at opposite sides of the microfluidic channel and are electrically coupled with each other via the fluid contained in the channel. This coupling can be of resistive- or capacitive nature but more generally, with AC coupling it has a combined resistive-capacitive character. In the embodiment of Fig. 6, the two excitation electrodes 5a and 5c belonging to the group of detection electrodes, are connected to the same AC potential: voltage sources generating voltage in the range of 0.001-50 V and the frequency of 10 kHz- 200 MHz.

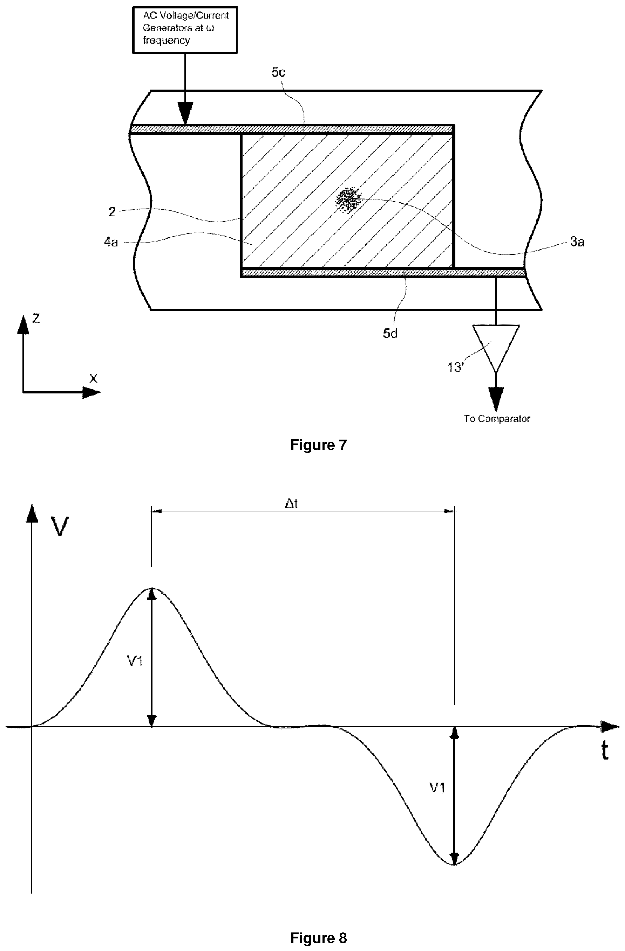

The positions of the electrodes are more clearly shown in Fig. 7 that is a cross-section of Fig. 6 through the line A-A' along a vertical plane perpendicular to the flow direction, i.e. in the ZX plane of Fig. 6. The other two detection electrodes, signal electrodes 5b and 5d, are electrically connected to two pre-amplifiers: pre-amplifier 13 and pre-amplifier 13'. In the simplest configuration, the two signal electrodes have identical dimensions, identical areas of contact with the interior of the common microfluidic channel, and are positioned at the same distance from the flow of the sample fluid 3a within the cross-section of the microfluidic channel, and the gains of the two pre-amplifiers are also identical. The signals from the preamplifiers are sent to ADCs and then digitised signals are processed using phase-sensitive detector/demodulator/lock-in amplifier.

The detection electrodes 5a and 5b detect the change in AC complex impedance between them (between the excitation electrode and signal electrode) resulting from the particles/cells passing through the detection area in between the excitation electrode/electrodes and the signal electrode/electrodes.

Once a particle/cell passes in between a pair of detection electrodes, it obstructs the electric coupling between the excitation electrode and the signal electrode. This changes the voltage induced in the signal electrode. The amplitude and the phase of the voltage induced in the signal electrodes depends on the properties of particle/cell and therefore can be used as a key indicator in identifying the particle (cell) or the subset to which the particle/cell belongs. The detection of this signal is done using the method of demodulation with the help of the lock-in or phase sensitive detection as described in the state of the art section of this document.

The identification of the particle/cell position and alignment should be done in real time within the same time interval as the measurements using the demodulation of the AC impedance change described above.

In order to improve signal-to-noise ratio of the signals induced in the signal electrodes, it is beneficial to use differential amplifiers. For this, a single signal electrode is replaced by two electrodes: signal electrodes 5b and 5d. These are connected to inputs of pre-amplifiers: pre-amplifiers 13 and 13'. The outputs of the pre-amplifiers are connected to a comparator/differential amplifier 14 as shown in Fig. 6. The excitation electrode is also separated into two electrodes 5a and 5c; and these are positioned opposite their respective signal electrodes 5b, 5d. In this case the signals from the pre-amplifiers 13 and 13' are introduced as inputs into the differential amplifier/comparator 14. The output from the pre-amplifiers is digitised by analogue-to-digital converter ADC1.

The comparator/differential amplifier 14 amplifies the difference between two inputs which are the outputs of the pre-amplifiers 13 and 13'. The output from the comparator is coupled to a lock-in amplifier or demodulator/phase sensitive detector capable of measuring the signal at the frequency w of the AC voltage source connected to the two excitation electrodes. The generator connected to the excitation electrodes is not shown in Fig. 6. Let us consider the operation by referring to one pair of excitation electrodes (5a and 5c) and one pair of signal electrodes (5b and 5d). In the absence of any particles/cells in the microfluidic channel, the output from the comparator is set to zero if the outputs of the pre-amplifiers 13 and 13' are set accurately to compensate for each other.

Once the particle/cell passes in between the excitation electrode and the signal electrode, it will induce signal in the comparator at the frequency w of the generator connected to the excitation electrodes. The typical amplitude of the output signal from the comparator is shown in Fig. 8. The signal will appear as a wave of one polarity followed by the wave of the opposite polarity and the same magnitude. This shape of the signal is due to the cell/particle passing first in front of the signal electrode connected to the pre-amplifier 13' and inducing signal of amplitude e.g. V1 that is introduced into the comparator with the positive polarity and then passing in front of the second signal electrode connected to the pre-amplifier 13 inducing rather the same signal V1 that is introduced into the comparator with a negative polarity. The time delay $\Delta t$ between the maxima/minima of first hump (peak) and the second one is indicative of the flow velocity in the channel as it is equal to the time required for the same cell/particle to travel from the position in front of the first sensing electrode to the same position in front of the second one. The detection of this signal is done using the method of demodulation with the help of the lock-in amplifier or phase sensitive detection. The amplitude and the phase of the signal V1 are indicative of the characteristics of the particle (cell) and therefore can be used as a key indicator in identifying the particle (cell) or the subset to which the particle (cell) belongs. Fig. 6 does not clearly show the difference between the excitation electrodes 5a and 5c on one hand and the signal electrodes 5b and 5d on the other hand. The excitation electrodes and the signal electrodes have the same size and positioned one underneath the other and therefore are not distinguishable from a top view as shown in Fig. 6.

Once the particle/cell is identified/measured in the detection area, it is possible to determine the time required for it to travel from the detection area to the separation area. In Fig. 6, this distance is marked as L. Exact definition of the distance L is embodiment-dependent. In Fig. 6 this is the distance from the centre of the first detection electrode to the beginning of the separation electrode. The flow velocity is normally well known in the in the microfluidic chip. There are several methods of detecting it and one of these methods is described above in relation to Fig. 8. The flow velocity $V_{flow}$ is equal to $V_{flow}=l1/\Delta t$ where $\Delta t$ is the time interval between the two maxima/minima of the peaks of Fig. 8, and l1 is the distance between the centres of the two subsequent detection electrodes (Fig. 6). Therefore, the control circuit can establish the time required for the particle/cell to travel from the detection area to the separation area. With reference to Fig. 6, if the flow velocity is $V_{flow}$, then the time required for the particle/cell to arrive to the separation area is $\Delta t=L/V_{flow}$ measured from the moment of the first peak shown in Fig. 8. Therefore, in order to direct the identified particle/cell into the correct secondary channel, we need to apply a pulse of voltage to the separation electrodes 16a and 16c at time interval $\Delta t$ after detection of the first peak in the detection area. There may be some corrections to be introduced to take into the account the time processing the signal in the detection circuit. We will not expand this in further details as this is clear to those skilled in the art of signal processing. In Fig. 6 the separation voltage pulse is shown applied to the separation electrode 16a while the separation electrode 16c is shown connected to the ground potential. We discussed earlier with reference to the previous figures that there are many other options of such a connection.

As will be appreciated by those skilled in the art of electronics, the supply of the signal from the AC voltage generator into demodulator, lock-in amplifier/phase sensitive detector as the frequency reference, is important. This is not shown for clarity of the figures.

In a typical embodiment the width of the excitation and signal electrodes is in the range of 0.05 mm to 50 mm. The typical width of the separation electrodes is also in the same range of dimensions.

The detection circuit given in Fig. 6 is just one possible embodiment. This invention can be practiced regardless of the detection method. This can be practiced with numerous other varieties of the detection based on AC impedance measurements. This can also be practiced in conjunction with optical detection, based on scattering of light by the particles/cells, optical fluorescence detection and indeed other methods of detection. The time interval from the moment of detecting/identifying the particle/cell to the moment of actuating the separation electrodes should preferably be calculated using the known distance from the detection element to the separation area and the using the known flow velocity. If required, one may have to add correction to take into account the delay time for the operation of the detection circuit.

In relation to the frequency of the voltage source/sources, the signal induced in the signal electrodes by cells/particles passing in front of the electrodes is due to conductance change in the space between the excitations and sensing electrodes and also due to permittivity change in the same space. It should be appreciated that the dielectric permittivity $\varepsilon(f)$ of a typical cell is frequency f dependent, and its conductance properties are also frequency dependent. These properties may also depend on other external factors such as pH liquid or temperature as the internal properties of the cells are affected by the conditions outside the cell. The conductance of the liquid bi-layer along the cell surface also depends on the pH of the liquid carrying the cells and its internal structure. It is preferable to choose the AC frequency f of the voltage energising the excitation electrodes such that the dielectric permittivity $\varepsilon$ at this frequency is significantly different from that of the liquid where the cells are placed. The dielectric permittivity is a complex function that has two components: the real Re $\varepsilon(f)$ and imaginary Im $\varepsilon(f)$ ones, both of these being functions of frequency. The same applies to conductance. It is preferable to operate the excitation electrodes at the frequency where there is significant difference between the conductance of the particles/cells and that of the liquid carrying these. Such a difference is responsible for the magnitude of the signal induced in the signal electrodes. Since cells have internal fine structure with elements of the structure having their own different dependencies of permittivity, there is generally a rather broad window of frequencies where the signal from the cells/particles can be readily detected. The frequency w is usually selected in such a window by tuning the frequency to optimise the value of the signal induced in the signal electrodes.

The walls of the microfluidic common microfluidic channel could be made of plastic, glass or other materials, e.g. silicon. The walls of the common microfluidic channel are indicated on Fig. 6 and Fig. 7 with numeral 2. The detection and the separation electrodes are made of metal, these could be e.g. gold, copper, silver electrodes or these could also be made out of other metals that have special bio-compatibility properties and enhanced resistance to corrosion such as palladium, platinum, tantalum, titanium, etc. The thickness of the electrodes is in the range 0.1-10 micrometers. The electrodes could be also be composed of several layers, for example a sub-layer of metal A serving the purpose of enhanced adhesion to the walls of the channel followed by a layer B deposited on the surface of the layer A. These details are well appreciated by those skilled in the art of making film electrodes for electronic and electrical applications. The detection electrodes are usually open to the interior of the common channel 2 across their width of the electrodes to make sound electric contact with the fluid inside the channel. The shielding electrodes 8a, 8b, 8c and 8d are shown in Fig. 6 but not shown in most other figures of the document for brevity. They also make electric contact with the interior of the common microfluidic channel. The configuration of the shielding electrodes and their positions are important as they change the distribution of the electric potential along the microfluidic channel and the pattern of current distribution between the excitation electrodes and the signal electrodes. This pattern is defined by the distribution of potentials in between the excitation and the signal electrodes and therefore, once the distribution of potential changes, the distribution of currents between the excitation electrode and the signal electrode, will also change.

As a brief note of the AC impedance detection, the signals received at each signal electrode in general are complex signals. They are characterised by amplitude and phase or otherwise by real and imaginary parts of the signal. Such complex response arises from a complex circuit in between the excitation and the signal electrode: there is resistive and capacitive coupling between the electrodes. The cells are also characterised by a dielectric constant that has real and imaginary parts. The imaginary part is responsible for the losses in the cell under the influence of the AC electric field. The liquid carrying particles/cells also has real and imaginary dielectric constant. For a given configuration of the electrodes and given characteristic of the liquid and the particles/cells, the importance of the phase contained in the signal is dependent on the excitation frequency w. There can be a window of frequencies where the phase characteristics of the signal collected from the signal electrode should not be neglected as it contains valuable information helping to identify the particles/cells. This information can be readily collected if the signal detection is done using phase-sensitive detector or lock-in amplifier.

The microfluidic device may also include means for sustaining and control of the fluid flow in the microfluidic channel, mixing the flows of the sample fluid and the sheath fluid, regulating the flows. The flow of fluids in the channels is sustained by a pump or multiple pumps or a pressure source/sources. This could be e.g. one or several UniGo pressure driven pumps from Cellix Limited, Dublin, Ireland. The pump/pumps are not shown in figures of this document for shortness as those skilled in the art of microfluidics are familiar with this aspect. There is a flow of the particle containing fluid 3a enveloped in the flow of the guidance fluid 4a. For shortness, we may call the particles-containing fluid also the sample fluid. We shall also refer to it as the liquid carrying particles/cells. Therefore, it is understood that the liquid carrying particles/cells and the particles/cells themselves form sample fluid. For example, this could be For example, this could be TRISA based buffer which is commonly used for holding sperm cells which might contain concentration of sperm cell from 0.1-100 million sperm cells per millilitre. The particle containing fluid is the fluid that contains the particles of interest. These could be organic or inorganic particles. These could also be alive or dead cells including mammalian cells, sperm cells, yeast cells, particles of biological and non-biological origin etc. In this embodiment, the particles-containing fluid stream (sample fluid) is located at the centre of the guidance fluid 4a flow, i.e. at the centre of the cross-section of the common microfluidic channel 2. Therefore, the guidance fluid 4a performs the function of the sheath fluid focussing the flow of the particle containing fluid into a tighter flow of reduced cross-section. One could construct other embodiments where the sample fluid is guided by the guidance fluid to be positioned not at the centre of the common microfluidic channel but e.g. at a corner of rectangular cross-section of the common microfluidic channel or along its wall and there are certain benefits of such a positioning. The guidance fluid also does not need to envelope the sample fluid all around but could be enveloped e.g. on two or three sides. All these embodiments are included in the present document. To obtain the focussing of the particles-containing fluid 3a by means of the guidance fluid, the microfluidic chip 1 comprises sample microfluidic channel 3 sustaining a flow of particles containing fluid and a guidance microfluidic channel or channels 4 sustaining a flow of guidance (sheath) fluid. Typically the flow rate of the guidance fluid is substantially greater than the flow rate of the sample fluid, i.e. greater by a factor of 2 to 100.

It will be appreciated by those familiar with hydrodynamic focussing that there is no sharp physical boundary between the sample fluid flow and the guidance fluid in the common channel. These two liquids gradually intermix by diffusion and under influence of other forces, along the common microfluidic channel as they move from the point of their mergence downstream. However, over the distance of 0.1-10 millimeters one could readily consider the flow as the flow of two fluids: the sample fluid enveloped by a guidance fluid. The guidance fluid 4a may be interchangeably called the sheath fluid in this document. The linear velocity of the liquid in the sample microfluidic channel and the guidance channel is in the range 0.01 meters/sec to 10 meters/sec. Those skilled in the art appreciate that the linear velocity varies strongly across the channel and normally is greatest at the centre of the channel at least for pressure-driven flows. This compression of the sample fluid by the sheath fluid through the laminar mixing of the streams in one common microfluidic channel, is known as hydrodynamic focussing. The focussed flow of the particles containing fluid gradually becomes defocussed due to the diffusive movement of the particles perpendicular to the flow direction. The detection area is located at short enough distance away from the point where the sample microfluidic channel merges with the guidance channels, at such a distance where the particles containing fluid still remains focussed at the centre of the common microfluidic channel 2. This distance could be in the range 20 to 2000micrometers.

We refer to particulates/particles throughout this document. This also includes clusters of particulatesparticles. The term cluster describes a small group of particles, for example 2-20 particles linked together by binding forces. For the purpose of this document we shall treat such clusters in the same way as single particles.

Figures of this document show excitation electrodes and signal electrodes making direct contact with the interior of the common microfluidic channel. This does not have to be always the case and one could construct embodiment where some or all of these electrodes are electrically insulated from the interior of the common microfluidic channel. This embodiment could more practical for operation at very high excitation frequencies, e.g. above 10 MHz.

The cross-section of the channel does not need to be rectangular or square. One could have circular, triangular, elliptical channel or indeed channels of various other cross-sections.

It is understood that cells addressed by the above description could be any live or dead cells, and non-mammalian mammalian cells, sperm cells, etc.

The use of hydrodynamic focussing is entirely optional. One may arrange the flow of sample fluid through the common microfluidic channel without any use of the sheath fluid.

All the embodiments shown in this document have separation electrodes positioned on two vertical walls. This is done so only for the clarity of the drawings. For example, with reference to Fig. 1, one or both of the separation electrodes could be positioned on the top of bottom wall of the common microfluidic channel or secondary channel, i.e. parallel to the plane YX. One could construct embodiment where some of the separation electrodes are positioned on the vertical wall (e.g. plane ZY of Fig. 1) and some other ones are positioned on the horizontal wall (e.g. plane YX of Fig. 1). The separation electrode does not need to cover the entire width or height of the wall of the channel. It could cover a fraction of the wall's width or height. It could also cover corner of two adjacent walls covered by separation electrodes across their entire width or height or partially.

Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

**Claims**

1. An apparatus for separation of particulates in a fluid into subsets of particulates, the apparatus comprising a microfluidic chip comprising:

   a microfluidic channel having a fluid inlet for receipt of a stream of particulate containing fluid,
   a detection zone disposed in the microfluidic channel and comprising a sensor configured to detect changes in the microfluidic channel corresponding to particulates passing the sensor; and
   a separation zone distal of the detection zone in which the microfluidic channel divides into at least two secondary microfluidic channels, the separation zone comprising two or more separation electrodes including at least one separation electrode disposed in electrical contact with an interior of the microfluidic channel and at least one further separation electrode disposed in electrical contact with an interior of the microfluidic channel or one of the secondary microfluidic channels,

   whereby the pair of separation electrodes are configured to pass a pulse of electrical current through the separation zone of the microfluidic channel.

2. An apparatus according to Claim 1 including a voltage/current generator electrically connected to one or more of the separation electrodes and configured to supply a voltage/current pulse to one or more of separation electrodes such that during use an electrical current passes between the electrodes.

3. An apparatus according to Claim 2 in which the voltage/current generator is configured to apply a voltage pulse to the separation electrodes in the range of 0.01-50 V of either positive or negative polarity.

4. An apparatus according to Claim 2 or 3 in which the voltage/current generator is configured to apply a voltage pulse to the separation electrode with the duration in the range of 1 microsecond to 50 milliseconds.

5.  An apparatus according to any of Claims 2 to 4 including a processor operatively connected to the sensor and the or each voltage/current generator and configured to receive signals from the sensor in the detection zone and actuate the or each voltage/current generator in response to signals received from the sensor.

6.  An apparatus as claimed in Claim 5 including two electrodes in the microfluidic channel and an electrode in each of the two secondary microfluidic channels.

7.  An apparatus as claimed in any preceding Claim in which the sensor in the detection zone is configured to measure AC impedance change between a set of detection electrodes positioned in the microfluidic channel corresponding to particulates obstructing the electric path between the detection electrodes.

8.  An apparatus as claimed in any of Claims 1 to 7 in which the sensor is configured to make optical measurements of the light scattered by the particulates or of the fluorescence signal from the particulates passing along the detection zone of the microfluidic channel.

9.  An apparatus as claimed in any preceding Claim including a hydrodynamic focussing mechanism configured to hydrodynamically focus the stream of particulate containing fluid to provide a focussed sample fluid within a guidance/sheath fluid.

10. An apparatus as claimed in any preceding Claim in which the cross sectional area of the secondary microfluidic channels is greater than the cross sectional area of the microfluidic channel.

11. A method to separate particulates in a particulate containing fluid, which method employs an apparatus according to any of Claims 1 to 10, the method comprising the steps of passing a particulate containing fluid along the microfluidic channel, employing the sensor to detect a change in the microfluidic channel corresponding to a particulate passing the sensor in the detection zone, and actuation of the separating electrodes in the separation zone in response to the change detected in the microfluidic channel to pass a pulse of current localised in the separation area and deflect the particulate into one of the secondary microfluidic channels.

12. A method according to Claim 11 in which the particulates are cells, and in which the method separates the cells according to a phenotype or in which the particulates are semen cells, and in which the method separates the cells according to a sex.

13. A method according to any of Claims 11 to 12 in which the apparatus comprises two pairs of separating electrodes including two electrodes in the microfluidic channel and an electrode in each of the two secondary microfluidic channels, wherein the two electrodes in the microfluidic channel are connected to a fixed potential and one of the electrodes in the secondary microfluidic channel is energised with a positive voltage and one of the electrodes in the secondary microfluidic channel is energised with a negative voltage.

14. A method according to any of Claims 11 to 13, in which the apparatus comprises two pairs of detection electrodes, and in which the method includes a step of determining the speed of particulate fluid in the microfluidic channel by detecting the time it takes a particulate to pass from a first pair of detection electrodes to a second pair of detection electrode.

15. A method according to Claim 11, in which the determined speed of the particulate containing fluid is correlated with a distance between the first pair of detection electrodes and the separation electrodes to calculate a time delay between (a) detecting a change in the microfluidic channel associated with a particulate passing the detection electrode and (b) actuation of the separating electrodes to deflect the particulate into one of the secondary microfluidic channels.

Figure 1

**Figure 2**

**Figure 3**

**Figure 4**

**Figure 5**

Figure 6

Figure 7

Figure 8

16

17a

4a

16b

3a

16c

Flow Direction

α3

α1

17b

2

16a

16d

17c

Z

Y

X

**Figure 9**

16

17b

W

W1

Flow Direction

W2

2

17b

Z

Y

X

**Figure 10a**

EP 3 418 717 A1

16
17a

W1
α1
W
Flow Direction
α2
W2
17b

2

Z
Y
X

Figure 10b

16
17a  16b
16c
Flow Direction
16a
2
17b  16d

Z
Y
X

Figure 11

26

16

17a

Flow Direction

2

17b

Z

Y

X

Figure 12

Europäisches
Patentamt

European
Patent Office

Office européen
des brevets

# EUROPEAN SEARCH REPORT

Application Number

EP 17 17 7624

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/128941 A1 (SADRI AMIR [CA] ET AL) 11 May 2017 (2017-05-11) | 1-5,7-12 | INV. G01N15/10 |
| Y | * paragraph [0033] * <br> * figure 19 * <br> * paragraph [0092] - paragraph [0094] * <br> * figure 9 * <br> * paragraph [0140] * <br> * paragraph [0040] * <br> * paragraph [0310] * <br> * paragraph [0102] * <br> * paragraph [0107] * <br> ----- | 6,13-15 | C12N5/071 B01L3/00 <br><br> ADD. G01N15/14 |
| T | 29.7: Displacement Current and Maxwell's Equations: <br> In: Young, H.D. & Freedman, R.A.: "University Physics 11th edition", 31 December 2004 (2004-12-31), Pearson Education, San Francisco, XP002773009, ISBN: 0-8053-8684-X pages 1128-1129, <br> * page 1128 - page 1129 * <br> ----- | 1-15 | |
| X | EP 2 508 253 A1 (JAPAN SCIENCE & TECH AGENCY [JP]) 10 October 2012 (2012-10-10) | 1-5,7-12 | TECHNICAL FIELDS SEARCHED (IPC) <br><br> G01N |
| Y | * paragraph [0012] - paragraph [0014] * <br> * paragraph [0032] * <br> ----- | 6,13-15 | C12N B01L |

The present search report has been drawn up for all claims

| Place of search | Date of completion of the search | Examiner |
|---|---|---|
| The Hague | 17 August 2017 | Mensink, Rob |

EPO FORM 1503 03.82 (P04C01)

# EP 3 418 717 A1

## ANNEX TO THE EUROPEAN SEARCH REPORT
## ON EUROPEAN PATENT APPLICATION NO.

EP 17 17 7624

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2017

| Patent document cited in search report | | Publication date | Patent family member(s) | | Publication date |
|---|---|---|---|---|---|
| US 2017128941 | A1 | 11-05-2017 | NONE | | |
| EP 2508253 | A1 | 10-10-2012 | CN | 102725060 A | 10-10-2012 |
| | | | EP | 2508253 A1 | 10-10-2012 |
| | | | JP | 4932066 B2 | 16-05-2012 |
| | | | JP | 5260763 B2 | 14-08-2013 |
| | | | JP | 5697112 B2 | 08-04-2015 |
| | | | JP | 2012137492 A | 19-07-2012 |
| | | | JP | 2013215725 A | 24-10-2013 |
| | | | JP | WO2011067961 A1 | 18-04-2013 |
| | | | KR | 20120096012 A | 29-08-2012 |
| | | | US | 2012298511 A1 | 29-11-2012 |
| | | | WO | 2011067961 A1 | 09-06-2011 |

EPO FORM P0459

For more details about this annex : see Official Journal of the European Patent Office, No. 12/82

29

# EP 3 418 717 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003107099 A
- WO 2008125081 A1
- US 20090283148 A1, Shinoda
- US 7294249 B, S. Gawad, M. Wuethrich, P. Renaud

### Non-patent literature cited in the description

- **S. GAWAD ; L. SCHILD ; P.H. RENAUD.** Micromachined impedance spectroscopy flow cytometer for cell analysis and particle sizing. *Lab. Chip.,* 2001, vol. 1, 76-82
- **MIKAEL EVANDER.** Microfluidic impedance cytometer for platelet analysis. *Lab on a chip,* 2013, vol. 13
- **R. RODRIGUEZ-TRUJILLO ; C. MILLS ; J. SAMITIER ; G. GOMILA.** *Microfluid. Nanofluid,* 2007, vol. 3, 171
- **P.WALSH ; E. WALSH ; M. DAVIES.** *Int. J. Heat Fluid Flow,* 2007, vol. 28, 44
- **R. SCOTT ; P. SETHU ; C.K. HARNETT.** Three-dimensional hydrodynamic focussing in a microfluidic Coulter counter. *Rev. Sci. Instruments,* 2008, vol. 79, 046104
- **YU-JUI CHIU ; S.H. CHO ; Z. MEI ; V. LIEN ; T.F. WU ; Y.H. LO.** Universally applicable three-dimensional hydrodynamic microfluidic flow focussing. *Lab Chip,* 2013, vol. 13, 1803
- **D.PARKS ; B. SAHAF ; O.PEREZ ; M. ROEDERER ; L.A. HERZENBERG.** *Clinical Chemistry,* 2002, vol. 48, 1819-1827
- **W.A. BONNER ; H.R. HULETT ; R.G. SWEET ; L.A. HERZENBERG.** *Review of Scientific Instruments,* 1972, vol. 43, 404-409
- **J. VOLDMAN.** *Annual Review of Biomedical Engineering,* 2006, vol. 8, 425-454
- **K. TAKAHASHI ; A. HATTORI ; I. SUZUKI ; T. ICHIKI ; K. YASUDA.** *Journal of Nanobiotechnology,* 2004, vol. 2
- **F. GUO ; X-H JI ; K. LIU ; R-X. HE ; L-B. ZHAO ; Z-X GUO ; W. LIU ; S-S GUO ; X-Z. ZHAO.** *Applied Physics Letters,* 2010, vol. 96, 193701
- **M.P. HUGHES.** Strategies for dielectrophoretic separation in laboratory on chip format. *Electrophoresis,* 2002, vol. 23, 2569-2582
- **B.H. LAPIZCO-ENCINAS ; B.A. SIMMONS ; E.B. CUMMINGS ; Y. FINTCHENKO.** Dielectrophoretic concentration and separation of live and dead bacteria in an array of insulators. *Anal Chem,* 2004, vol. 76, 1571-1579
- **N. LEWPIRIYAWONG ; K. KANDASWAMY ; C. YANG ; V. IVANOV ; R. STOCKER.** Microfluidic characterization and continuous separation of cells and particles using conducting poly(dimethyl siloxane) electrode induced alternating current-dielectrophoresis. *Anal. Chem.,* 2011, vol. 83, 9579-9585
- **H. SHAFIEE ; M.B. SANO ; E.A. HENSLEE ; J.L. CALDWELL ; R.V. DAVALOS.** Selective isolation of live/dead cells using contactless dielectrophoresis (cDEP). *Lab Chip,* 2010, vol. 10, 438-445
- **H. LI ; R. BASHIR.** Dielectrophoretic separation and manipulation of live and heat treated cell of Listeria on microfabricated devices with integrated electrodes. *Sensors and Actuators B,* 2002, vol. 86, 215-221
- **J.J. AGRESTI ; E. ANTIPOV ; A.R. ABATE ; K. AHN ; A.C. ROWAT ; J.C. BARET ; M. MARQUEZ ; A.M. KLIBANOV ; A.D. GRIFFITTHS ; D.A. WEITZ.** *Proceedings of the National Academy of Sciences of the United States of America,* 2010, vol. 107, 4004-4009
- **L. MAZUTIS ; J. GILBERT ; W.L. UNG ; D.A. WEITZ ; A.D. GRIFFITHS ; J.A. HEYMAN.** *Nature Protocols,* 2013, vol. 8, 870-891
- **A.Y. FU ; C. SPENCE ; A. SCHERER ; F.H. ARNOLD ; S.R. QUAKE.** *Nature Biotechnology,* 1999, vol. 17, 1109-1111
- **P.S. DITTRICH ; P. SCHWILLE.** *Analytical Chemistry,* 2003, vol. 75, 5767-5774
- **T.FRANKE ; S. BRAUNMULLER ; L. SCHMID ; A. WIXFORTH ; D.A. WEITZ.** *Lab Chip.,* 2010, vol. 10, 789-794
- **S.C. LIN ; X. MAO ; T.J. HUANG.** *Lab. Chip.,* 2011, vol. 11, 1280-1285
- **S.C. LIN ; X. MAO ; T.J. HUANG.** *Lab. Chip.,* 2012, vol. 12, 2766-2770
- **A. JONAS ; P. ZEMANEK.** *Electrophoresis,* 2008, vol. 29, 4813-4851